# EUROPEAN PATENT APPLICATION

(11) **EP 3 791 885 A1**
(43) Date of publication of application: **17.03.2021**
(21) Application number: 19382792.0
(22) Date of filing: 13.09.2019
(51) Int. Cl.: A61K 31/77, A61P 1/18

(54) **POLYETHYLENE GLYCOL FOR USE IN THE PREVENTION OF ABDOMINAL INFLAMMATORY DISEASES AND/OR ASSOCIATED DISEASES**

(71) Applicant: Consejo Superior de Investigaciones Científicas (CSIC), 28006 Madrid (ES); Net Abraham, Marc, 08003 Barcelona (ES); Olive, Guillaume, 13012 Marseille (FR); Lopez, Alexandre, 69160 Tassin la Demi Lune (FR)
(72) Inventor: NET ABRAHAM, Marc, 08003 Barcelona (ES); OLIVE, Guillaume, 13012 Marseille (FR); LOPEZ, Alexandre, 69160 Tassin La Demi Lune (FR); ROSELLÓ CATAFAU, Joan, 08036 Barcelona (ES)
(74) Representative: Pons

(57) **Abstract**

The invention relates to polyethylene glycol (PEG), particularly PEG35, for use in the prevention of abdominal inflammatory diseases and/or associated diseases, particularly for the prevention of acute necrotizing pancreatitis (ANP) and/or associated lung injury. The invention also relates to a pharmaceutical composition thereof.

## Description

The invention relates to polyethylene glycol (PEG), particularly PEG35, for use in the prevention of abdominal inflammatory diseases and/or associated diseases, particularly for the prevention of acute necrotizing pancreatitis (ANP) and/or associated lung injury. The invention also relates to a pharmaceutical composition thereof.

### BACKGROUND ART

Acute pancreatitis, an inflammatory disorder of the pancreas, is the main cause of hospitalizations related to gastrointestinal diseases and the fifth most common cause of death in hospitals (Lankisch PG, Apte M, Banks PA: Acute pancreatitis. Lancet 2015; 386:85-96). The revised universal classification system of acute pancreatitis distinguishes between two different forms (Banks PA, Bollen TL, Dervenis C, et al: Acute Pancreatitis Classification Working G: Classification of acute pancreatitis--2012: revision of the Atlanta classification and definitions by international consensus. Gut 2013; 62:102-11): interstitial edematous, and necrotizing. Interstitial edematous acute pancreatitis represents 80-90% of clinical cases and does not present local or systemic complications. However, up to 20% of patients develop acute necrotizing pancreatitis (ANP), the more aggressive form, which is associated with both local and systemic complications and a high risk of mortality; lung failure is the main contributing factor to early death within the first week after admission (Pastor CM, Matthay MA, Frossard JL: Pancreatitis-associated acute lung injury: new insights. Chest 2003; 124:2341-51). At present, no specific medical treatment for ANP exists; the management of the disease is mainly supportive and targeted to prevent and treat systemic complications.

Polyethylene glycols (PEGs) are non-immunogenic, non-toxic and water-soluble polymers composed of repeating units of ethylene glycol (Harris JM, Chess RB: Effect of pegylation on pharmaceuticals. Nat Rev Drug Discov 2003; 2:214-21). One of their most positive characteristics is their low toxicity regardless of the route of administration (Fruijtier-Polloth C: Safety assessment on polyethylene glycols (PEGs) and their derivatives as used in cosmetic products. Toxicology 2005; 214:1-38). In fact, PEG is currently the only water-soluble polymer that is widely accepted and approved by the Food and Drug Administration for use in food, cosmetics and pharmaceuticals (Veronese FM, Pasut G: PEGylation, successful approach to drug delivery. Drug Discov Today 2005; 10:1451-8). Besides, in clinical settings, PEGs are used as additives to organ preservation solutions to attenuate the damage associated with cold ischemia-reperfusion of kidney, pancreas and liver transplantation (Pasut G, Panisello A, Folch-Puy E, et al: Polyethylene glycols: An effective strategy for limiting liver ischemia reperfusion injury. World J Gastroenterol 2016; 22:6501-8.).

In recent years, several experimental studies have focused on the protective effects of PEGs. In lung endothelial cells, treatment with 15-20 kDa molecular weight PEG (PEG 15-20) was found to enhance cell function by activating endothelial cell-barrier signal transduction pathways and contributing to cytoskeleton reorganization (Chiang ET, Camp SM, Dudek SM, et al: Protective effects of high-molecular weight polyethylene glycol (PEG) in human lung endothelial cell barrier regulation: role of actin cytoskeletal rearrangement. Microvasc Res 2009; 77:174-86). In addition, pre-treatment with PEG 15-20 in cultured ventricular myocytes subjected to hypoxia-reoxygenation reduced oxidative stress and apoptosis and increased cell survival (Malhotra R, Valuckaite V, Staron ML, et al: High-molecular-weight polyethylene glycol protects cardiac myocytes from hypoxia- and reoxygenation-induced cell death and preserves ventricular function. Am J Physiol Heart Circ Physiol 2011; 300:H1733-42). The protective effects of PEGs have also been reported in *in vivo* models, although the literature on the context of inflammatory processes is scarce. In a murine model of lethal gut-derived sepsis therapeutic administration of low molecular weight PEG provided protection against bacterial infections and reduced mortality (Ackland GL, Gutierrez Del Arroyo A, Yao ST, et al: Low-molecular-weight polyethylene glycol improves survival in experimental sepsis. Crit Care Med 2010; 38:629-36). Likewise, prophylactic oral administration of PEG 4000 in experimental colitis strengthened the epithelial barrier and reduced inflammation of the colon (Videla S, Lugea A, Vilaseca J, et al: Polyethylene glycol enhances colonic barrier function and ameliorates experimental colitis in rats. Int J Colorectal Dis 2007; 22:571-80). Another study found that coating the peritoneal surfaces of the rat with PEG was a highly effective measure to attenuate the number of leukocytes in a model of post-traumatic inflammation (Nagelschmidt M, Minor T, Saad S: Polyethylene glycol 4000 attenuates adhesion formation in rats by suppression of peritoneal inflammation and collagen incorporation. Am J Surg 1998; 176:76-80). Lastly, in colon carcinogenesis, animals receiving a diet with PEG 8000 reduced colonic inflammation (Karlsson PC, Hughes R, Rafter JJ, et al: Polyethylene glycol reduces inflammation and aberrant crypt foci in carcinogen-initiated rats. Cancer Lett. 2005 Jun 8;223(2):203-9.).

The benefits of 35-kDa molecular weight PEG (PEG35) have been explored in experimental models of cold and warm liver ischemia/reperfusion in the rat. The addition of PEG35 to the organ preservation solution decreased liver graft vulnerability to cold ischemia-reperfusion injury (Zaouali MA, Bejaoui M, Calvo M, et al: Polyethylene glycol rinse solution: an effective way to prevent ischemia-reperfusion injury. World J Gastroenterol 2014; 20:16203-14). Furthermore, prophylactic intravenous administration of PEG35 to rats reduced ischemia/reperfusion-induced hepatic injury associated with the preservation of the mitochondrial status, cytoskeleton protection and the induction of cyto-protective signaling pathways (Bejaoui M, Pantazi E, Calvo M, et al: Polyethylene Glycol Preconditioning: An Effective Strategy to Prevent Liver Ischemia Reperfusion Injury. Oxid Med Cell Longev 2016; 2016:9096549). Intravital microscopy studies demonstrated the location of PEG35 adsorbed in the liver vascular bed after ischemia/reperfusion.

In view of the numerous benefits of PEGs just described, and of the fact that they are water-soluble and above all non-toxic, it would be desirable to explore its protective effects in pancreatitis and associated diseases.

### SUMMARY OF THE INVENTION

A first aspect of the present invention relates to polyethylene glycol (PEG), preferably to PEG of at least 5 kDa of molecular weight, and more preferably to PEG35, for use in the prevention of abdominal inflammatory diseases and/or associated diseases, and preferably for use in the prevention of acute or chronic pancreatitis and/or associated diseases.

In another embodiment the invention relates to PEG, and preferably to PEG35, for the use as defined above, wherein the associated diseases are selected from local associated diseases and systemic associated diseases.

In another embodiment the invention relates to PEG, and preferably to PEG 35, for the use as defined above, wherein the local associated diseases are selected from fluid collection ascites, pancreatic pseudocyst, pancreatic necrosis, and infective pancreatic necrosis.

In another embodiment the invention relates to PEG, and preferably to PEG 35, for the use as defined above, wherein the systemic associated diseases are selected from lung injury, renal dysfunction, shock, liver dysfunction, gastrointestinal bleeding, colitis and thrombosis.

In another embodiment the invention relates to PEG, and preferably to PEG35, for use in the prevention of acute necrotizing pancreatitis (ANP) and/or associated lung injury.

In another embodiment the invention relates to PEG, and preferably to PEG35, for the use defined above, characterized in that it is administered by intravenous administration.

In another embodiment the invention relates to PEG, and preferably to PEG35, for use in the prevention of pancreatitis and one or more associated diseases, characterized in that is administered by intravenous administration.

In another embodiment the invention relates to PEG, and preferably to PEG35, for use in the prevention of ANP and associated lung injury, characterized in that is administered by intravenous administration.

In another embodiment the invention relates to PEG, and preferably, to PEG35, for the use defined above, wherein the dose of PEG administered to a subject in need thereof is from about 0.01 mg/Kg to about 100 mg/Kg of body weight per day, and preferably of 10 mg/kg of body weight per day.

In another embodiment the invention relates to PEG, and preferably to PEG35, for use in the prevention of pancreatitis and one or more associated diseases, wherein the dose of PEG administered to a subject in need thereof is from about 0.01 mg/Kg to about 100 mg/Kg of body weight per day, and preferably of 10 mg/kg of body weight per day.

In another embodiment the invention relates to PEG, and preferably to PEG35, for use in the prevention of ANP and/or associated lung injury, wherein the dose of PEG administered to a subject in need thereof is from about 0.01 mg/Kg to about 100 mg/Kg of body weight per day, and preferably of 10 mg/kg of body weight per day.

In another embodiment the invention relates to PEG, and preferably to PEG35, for use in the prevention of pancreatitis and one or more associated diseases, wherein the dose of PEG administered to a subject in need thereof is:
- intravenously administered; and
- from about 0.01 mg/Kg to about 100 mg/Kg of body weight per day, and preferably of 10 mg/kg of body weight per day

In another embodiment the invention relates to PEG, and preferably to PEG35, for use in the prevention of ANP and/or associated lung injury, wherein the dose of PEG administered to a subject in need thereof is:
- intravenously administered; and
- from about 0.01 mg/Kg to about 100 mg/Kg of body weight per day, and preferably of 10 mg/kg of body weight per day.

In another embodiment the invention relates to PEG, and preferably to PEG35, for the above defined use, wherein the dose of PEG administered to a subject in need thereof is a single or divided doses, and preferably in a single dose.

In another embodiment the invention relates to PEG, and preferably to PEG35, for the use defined above, wherein the dose of PEG administered to a subject in need thereof is a single or divided doses from about 0.01 mg/Kg to about 100 mg/Kg of body weight per day, preferably of 10 mg/Kg of body weight per day, and preferably in a single dose from about 0.01 mg/Kg to about 100 mg/Kg of body weight per day, and more preferably in a single dose of 10 mg/Kg of body weight per day.

In another embodiment the invention relates to PEG, and preferably to PEG35, for use in the prevention of pancreatitis and one or more associated diseases, wherein the dose of PEG administered to a subject in need thereof is a single or divided doses, and preferably in a single dose, from about 0.01 mg/Kg to about 100 mg/Kg of body weight per day, and preferably of 10 mg/Kg of body weight per day.

In another embodiment the invention relates to PEG, and preferably to PEG35, for use in the prevention of ANP and/or associated lung injury, wherein the dose of PEG administered to a subject in need thereof is a single or divided doses, and preferably in a single dose, from about 0.01 mg/Kg to about 100 mg/Kg of body weight per day, and preferably of 10 mg/Kg of body weight per day.

In another embodiment the invention relates to PEG, and preferably to PEG35, for use in the prevention of pancreatitis and one or more associated diseases, wherein the dose of PEG administered to a subject in need thereof is:
- intravenously administered; and
- in a single or divided doses, and preferably in a single dose, from about 0.01 mg/Kg to about 100 mg/Kg of body weight per day, and preferably of 10 mg/kg of body weight per day.

In another embodiment the invention relates to PEG, and preferably to PEG35, for use in the prevention of ANP and/or associated lung injury, wherein the dose of PEG administered to a subject in need thereof is:
- intravenously administered; and
- in a single or divided doses, and preferably in a single dose, from about 0.01 mg/Kg to about 100 mg/Kg of body weight per day, and preferably of 10 mg/kg of body weight per day.

Another aspect of the present invention relates to a pharmaceutical composition which comprises a therapeutically effective amount of PEG, and preferably of PEG35, and a pharmaceutically acceptable carrier for use in the prevention of pancreatitis and associated diseases, and preferably for use in the prevention of acute necrotizing pancreatitis (ANP) and/or associated lung injury.

The term "polyethylene glycol" (PEG) is a poly(ether) compound. PEG is also known as polyethylene oxide (PEO) or polyoxyethylene (POE). PEG may be classified depending on its molecular weight. Along present invention PEG preferably refers to high molecular weight PEG of at least 5 kDa of molecular weight. Accordingly, for example, PEG include among others "PEG35" which is polyethylene glycol of 35,000 Da or 35 kDa.

The term "pancreatitis" as used herein refers to an inflammation of the pancreas. Pancreatitis can be either acute or chronic. Acute pancreatitis generally develops suddenly, and chronic pancreatitis is a long-term condition, which typically develops after multiple episodes of acute pancreatitis. Acute pancreatitis distinguishes between two different forms: Interstitial edematous acute pancreatitis with no local or systemic complications and acute necrotizing pancreatitis (ANP), associated with both local and systemic complications and a high risk of mortality.

The term "associated injury" as used herein refers to associated local and systemic complications of pancreatitis. Local associated injury includes fluid collection, ascites, pancreatic pseudocyst, pancreatic necrosis, and infective pancreatic necrosis. Systemic associated injury includes lung injury, such as arterial hypoxia, atelectasis, pneumonia, pleural effusion, mediastinal abscess, respiratory failure and adult respiratory distress syndrome (ARDS). In addition, the inflammatory changes from the pancreas may extend to the kidneys, heart, liver, stomach, colon and splenic vein. This may result in renal dysfunction, shock, liver dysfunction, gastrointestinal bleeding, colitis and splenic vein thrombosis, respectively.

The term "prevention" as used herein refers to the prophylactic and therapeutic effect, that is, to any activity that avoids, delays the onset of or reduces the progression of a disease.

Injectable preparations, according to the present invention, for parenteral administration, comprise sterile solutions, suspensions or emulsions, in an aqueous or non-aqueous solvent. These compositions can also contain coadjuvants, such as wetting, emulsifying, dispersing agents and preservatives. They may be sterilized by any known method or prepared as sterile solid compositions which will be dissolved in water or any other sterile injectable medium immediately before use. It is also possible to start from sterile materials and keep them under these conditions throughout all the manufacturing process.

The dosage and frequency of doses will depend upon the nature and severity of the disease to be treated, the age, the general condition and body weight of the patient, as well as the particular compound administered and the route of administration, among other factors. A suitable dosage range is from about 0.01 mg/Kg to about 100 mg/Kg per day, and preferably a suitable dosage is of 10 mg/Kg per day, which can be administered as a single or divided doses, and preferably as a single dose.

As used herein the term "pharmaceutically acceptable" is meant that the carrier, diluent, excipients, and/or salt must be compatible with the other ingredients of the formulation, and not deleterious to the recipient thereof. "Pharmaceutically acceptable" also means that the compositions or dosage forms are suitable for use for an animal or human without excessive toxicity, irritation, allergic response, or other problem or complication, commensurate with a reasonable benefit/risk ratio.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings and sequence listing are provided by way of illustration and are not intended to be limiting of the present invention.

### BRIEF DESCRIPTION OF THE DRAWINGS

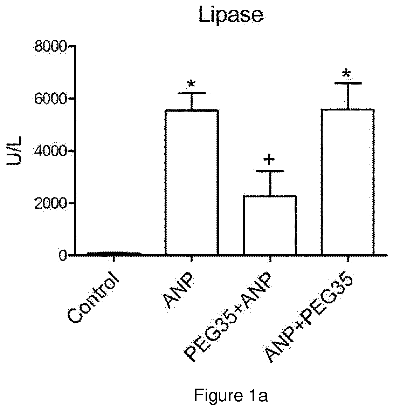

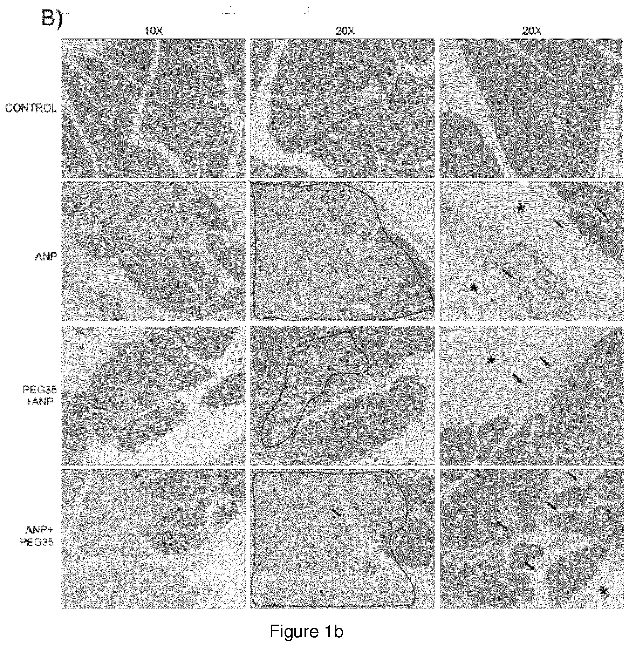

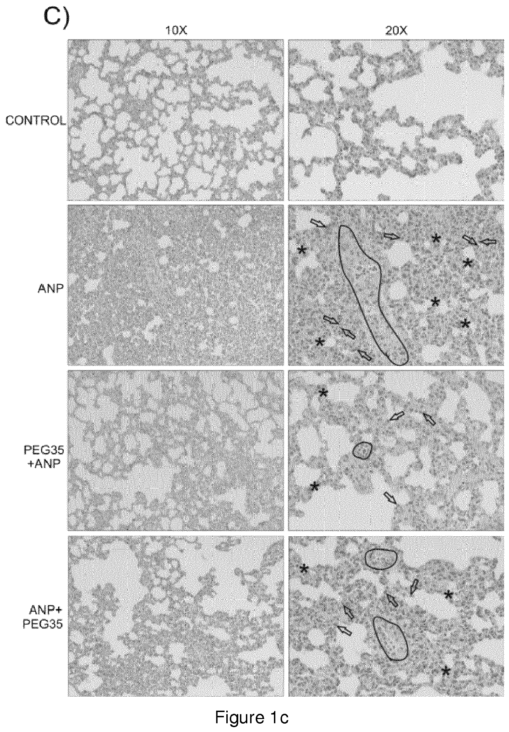

**Fig. 1****. Effect of PEG35 treatment on the severity and histological changes in experimental acute necrotizing pancreatitis and associated acute lung injury.** A) Plasma lipase levels in U/L. Bars represent mean values of each group ± SEM. **P* < 0.05 versus Control, +*P* < 0.05 versus ANP. ANP, Acute Necrotizing Pancreatitis. Each determination was carried out in triplicate. B) Representative images of hematoxylin and eosin-stained pancreatic sections for each experimental group. Control group showed normal pancreas structure. ANP and ANP+PEG35 groups presented large areas of necrosis (under area), infiltrated polymorphonuclear neutrophils (black arrows) and interstitial edema (*). Prophylactic administration of PEG35 significantly reduced these features. C) Representative images of hematoxylin and eosin-stained lung sections for each experimental group. Control group showed normal alveolar structure. In the ANP group, a marked alveolar septal thickening (indicated by an asterisk), leukocyte infiltration (indicated by empty arrows) and the presence of vessel neutrophils (under area) were seen. Both prophylactic and therapeutic PEG35 treatment normalized alveolar septal thickening and leukocyte infiltration. Images were acquired at 10X and 20X magnification. ANP, Acute Necrotizing Pancreatitis.

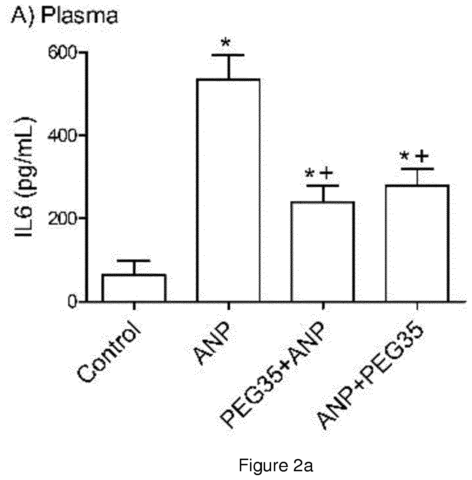

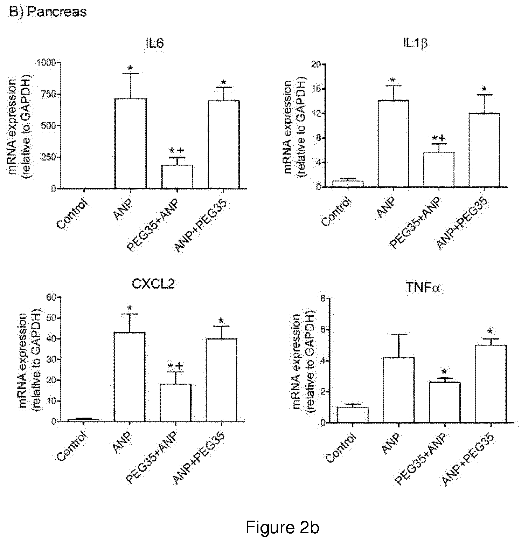

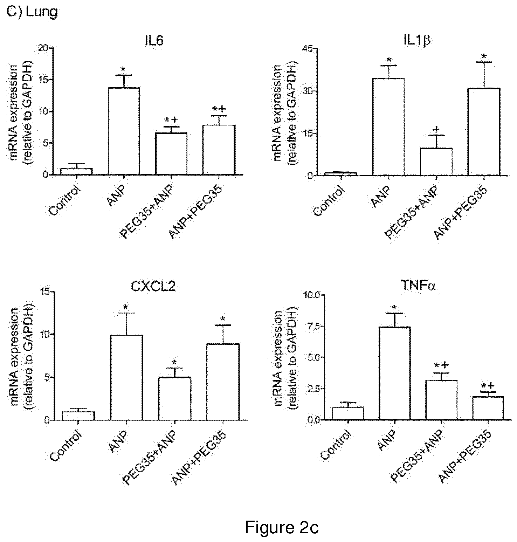

**Fig. 2****. Role of PEG35 on the modulation of pro-inflammatory cytokines and chemokines expression in sodium taurocholate acute necrotizing pancreatitis and associated acute lung injury.** A) IL6 expression levels in plasma B) Pancreatic tissue gene expression of IL6, IL1β, CXCL2 and TNFα by real-time qRT-PCR. C) Lung tissue gene expression of IL6, IL1β, CXCL2 and TNFα by real-time qRT-PCR. In all cases, mRNA induction levels were normalized to GAPDH mRNA expression. Bars represent mean values of each group ± SEM. **P* < 0.05 versus Control, ⁺*P* < 0.05 versus ANP. ANP, Acute Necrotizing Pancreatitis. Each determination was carried out in triplicate.

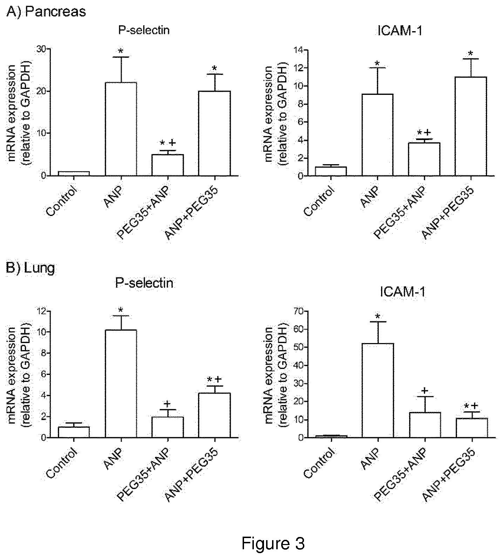

**Fig. 3****. Effects of PEG35 administration on the expression of adhesion molecules in acute necrotizing pancreatitis and associated acute lung injury.** A) Pancreatic tissue gene expression of P-selectin and ICAM-1 by real-time qRT-PCR. B) Lung tissue gene expression of P-selectin and ICAM-1 by real-time qRT-PCR. In all cases, mRNA induction levels were normalized to GAPDH mRNA expression. Bars represent mean values of each group ± SEM. **P* < 0.05 versus Control, **P* < 0.05 versus Control, +*P <* 0.05 versus ANP. ANP, Acute Necrotizing Pancreatitis. Each determination was carried out in triplicate.

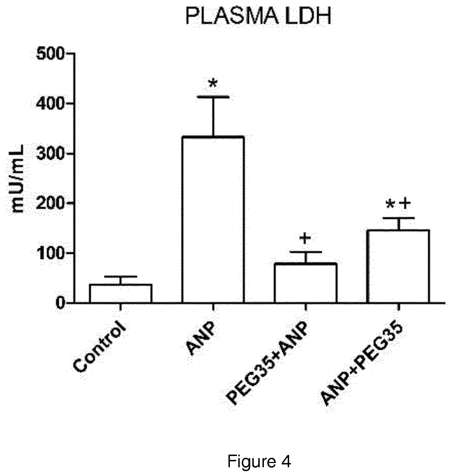

**Fig. 4****. Effects of PEG35 treatment on lactate dehydrogenase activity in plasma three hours after acute necrotizing pancreatitis induction.** Bars represent mean values of each group ± SEM. **P* < 0.05 versus Control, +*P* < 0.05 versus ANP. ANP, Acute Necrotizing Pancreatitis. Each determination was carried out in triplicate.

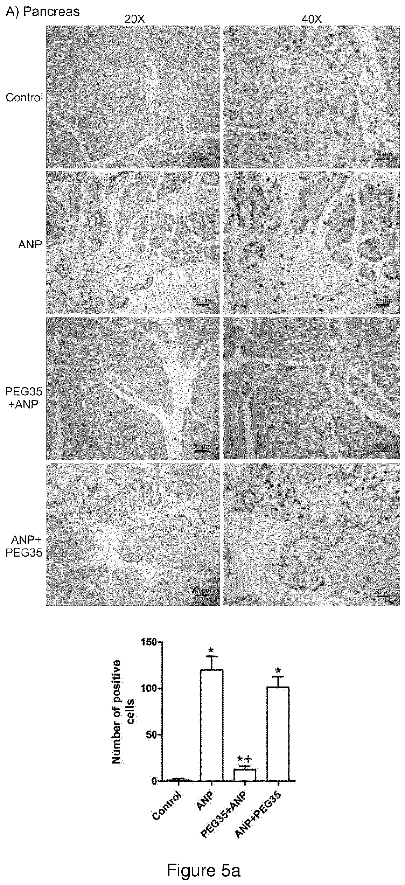

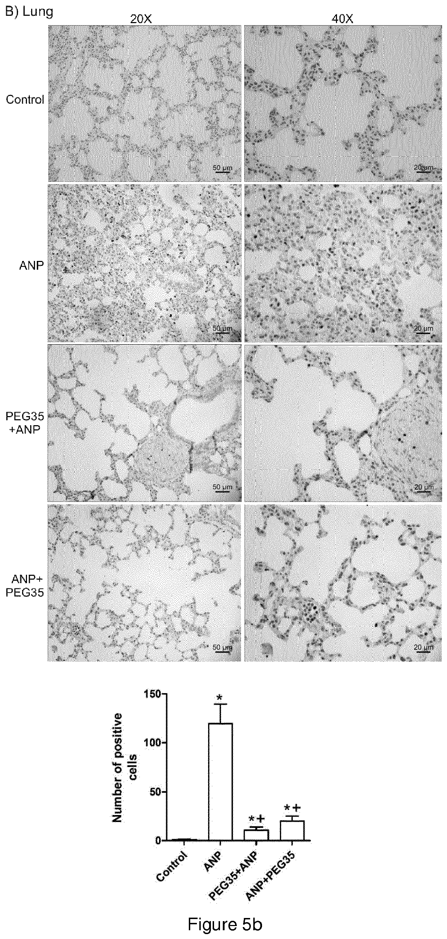

**Fig. 5****. Effects of PEG35 treatment on acute necrotizing pancreatitis-induced myeloperoxidase expression.** A) Up, Representative images of pancreatic sections stained with anti-MPO antibody (brown). Down, Pancreas MPO immunostaining quantification represented as number of positive cells. B) Up, Representative images of lung sections stained with anti-MPO antibody (brown). Down, Pulmonary MPO immunostaining quantification represented as number of positive cells. Images were acquired at 20X and 40X magnification. ANP, Acute Necrotizing Pancreatitis.

### EXAMPLES

### MATERIALS AND METHODS

### Experimental animals

Male Wistar rats weighting 200-250 g were housed in a controlled environment and fed with a standard laboratory pelleted formula (A04; Panlab, Barcelona, Spain) and tap water *ad libitum.* All procedures were conducted in accordance with European Union regulatory standards for animal experimentation (Directive 2010/63/EU on the protection of animals used for scientific purposes). The Ethical Committee for Animal Experimentation (CEEA, Directive 396/12, University of Barcelona) approved the animal experiments.

### Animal model of ANP

The rats were anesthetized with pentobarbital. After a midline laparotomy, the biliopancreatic duct was cannulated through the duodenum and the hepatic duct was clamped in order to prevent the flow of bile. The experimental model of ANP was induced in the rats (n=8) by retrograde injection of 5% sodium taurocholate in a volume of 0.1 ml/100 g body weight using a perfusion pump (Harvard Instruments, Edenbridge, UK). Control animals (n=8) received saline solution (NaCl 0.9%). This model represents the reference standard of biliary acute pancreatitis, the most common cause of ANP in humans. As previously reported, the infusion of this bile salt at 5% results in lung injury after 3 hours of induction. This lung failure is the main contributing factor to early death in patients with ANP (Folch E, Salas A, Panes J, et al: Role of P-selectin and ICAM-1 in pancreatitis-induced lung inflammation in rats: significance of oxidative stress. Ann Surg 1999; 230:792-8; discussion 8-9). PEG35 was administered intravenously in a single dose of 10mg/kg either prophylactically (10 minutes before ANP induction) or therapeutically (10 minutes after ANP induction) (n=8 for each group). Three hours after ANP induction, harvested heparinized blood was centrifuged, the plasma removed and stored at -80°C. Sections of the pancreas and lungs were fixed in 4% phosphate-buffered formalin. Freshly harvested tissue samples of pancreas and lungs were immediately frozen and stored at -80ºC or stored in RNAlater solution.

### Lipase activity

Plasma lipase activity was determined using a commercial turbidimetric assay kit from Randox (County Antrim, UK), according to the supplier's specifications. The decrease in turbidity was measured at 340 nm using an automated microplate reader (iEMS Reader MF; Labsystems, Helsinki, Finland). All samples were run in duplicate.

### Histopathological examination

Pancreatic and lung tissue were fixed in 10% buffered formalin and embedded in paraffin. Sections of 3µm thickness were mounted on glass slides. Slides were dewaxed and rehydrated before staining with hematoxylin and eosin. Then, a pathologist examined multiple randomly chosen microscopic fields from each experimental group in a blinded manner. Sections of pancreas tissue were scored for the severity of pancreatitis based on edema, leukocyte infiltration and necrosis. Histological evaluation of lung injury was assessed using a scale for interstitial and intra-alveolar leukocyte infiltration and alveolar septal thickening.

### IL6 immunoassay

Interleukin-6 (IL6) in plasma was measured using a commercially available ELISA Kit (R&D Systems, Minneapolis, MN, USA) in accordance with the manufacturer's instructions. The optical density was measured at 450 nm using an automated microplate reader (iEMS Reader MF; Labsystems, Helsinki, Finland). All samples were run in duplicate.

### Lactate dehydrogenase activity

Lactate dehydrogenase (LDH) was measured in samples of plasma using the Lactate Dehydrogenase Assay Kit (Abcam; Cambridge, UK). Changes in absorbance due to NAD+ formation were recorded at 450 nm at 37 °C using an automated microplate reader (iEMS Reader MF; Labsystems, Helsinki, Finland). All samples were run in duplicate.

### Real-time qRT-PCR

Total RNA from the pancreas and lungs was extracted with TRIzol reagent (Invitrogen, Carlsbad, CA, USA) according to the manufacturer's protocol. The amount of RNA was determined by measuring the absorbance at 260 and 280 nm. Reverse transcription was conducted on a 1 µg RNA sample using the iScript cDNA Synthesis Kit (Bio-Rad Laboratories, Hercules, CA, USA). Subsequent PCR amplification was conducted using SsoAdvanced™ Universal SYBR® Green Supermix (Bio-Rad Laboratories, Hercules, CA, USA). PCR primers for the detection of Interleukin 6 (IL6), Interleukin 1β (IL1β) and glyceraldehyde-3-phosphate dehydrogenase (GAPDH) were experimentally validated primers from BioRad (Hercules, CA, USA). PCR primers for Chemokine (C-X-C motif) ligand 2 (CXCL2), Tumor necrosis factor α (TNFα), P-selectin and Intercellular adhesion molecule-1 (ICAM-1) were designed with Primer3.0 plus (Untergasser A, Cutcutache I, Koressaar T, et al: Primer3--new capabilities and interfaces. Nucleic Acids Res 2012; 40:e115). The sequence were as follows: CXCL2 forward, 5'-TGCTCAAGACTCCAACCACTC-3' (SEQ ID NO: 1) and reverse, 5'-CACAACAACCCCTGTACCCTG-3' (SEQ ID NO: 2); TNFα forward, 5'-ATGGGCTCCCTCTCATCAGT-3' (SEQ ID NO: 3) and reverse, 5'-GCTTGGTGGTTTGCTACGAC-3' (SEQ ID NO: 4); P-Selectin forward, 5'-TCTCCTGCAACGAGGAGTTT-3' (SEQ ID NO: 5) and reverse, 5'-GGTGTCGACAGGACATTGTG-3' (SEQ ID NO: 6); and ICAM-1 forward, 5'-GAGCGACATTGGGGAAGACA-3' (SEQ ID NO: 7) and reverse, 5'-CACTCGCTCTGGGAACGAATA-3' (SEQ ID NO: 8). Reactions were carried out in duplicate and threshold cycle values were normalized to glyceraldehyde-3-phosphate dehydrogenase (GAPDH) gene expression. The ratio of the relative expression of target genes to GAPDH was calculated by the DCt formula.

### Immunohistochemistry

Pancreatic and lung tissue were fixed and embedded in paraffin slices. After quenching endogenous peroxidase activity, antigen retrieval was conducted by incubating samples with sodium citrate. Then, the tissue sections were incubated overnight with the myeloperoxidase antibody (1:100 dilution; reference ab9535 Abcam; Cambridge, UK). Sections were then incubated with the appropriate biotinylated secondary antibody followed by conjugated horseradish peroxidase-streptavidin. Slides were counterstained with hematoxylin and mounted. Images were taken with a Nikon Eclipse E1000 microscope (Nikon, Amsterdam, Netherlands) and analyzed using Cell^B imaging software (Olympus, Hamburg, Germany). The mean number of peroxidase-positive cells was counted in five randomly chosen microscopic high-power fields (20X) per animal in a blinded fashion.

### Statistical analysis

All data were exported into Graph Pad Prism 4 (GraphPad Software, Inc.) and were presented as means ± SEM. Statistical analyses were carried out by one-way analysis of variance (ANOVA), followed by Tukey's multiple comparison test to determine the significance between pairs. The minimal level of significance was considered at P < 0.05.

### RESULTS

### Effects of PEG35 on plasma lipase levels

Sodium taurocholate-induced ANP in rats was associated with significant increases in the plasma levels of lipase reflecting the degree of pancreatic injury in this experimental model (Figure 1A). This increase was significantly reduced in the rats pretreated with 10mg/kg of PEG35. In contrast, therapeutic PEG35 administration had no effect on the pancreatic injury associated with ANP.

### Prophylactic and therapeutic PEG35 reduced systemic tissue damage associated with ANP

Intraductal administration of 5% sodium taurocholate in the rats produced a severe hemorrhagic pancreatitis with large areas of interstitial edema, necrosis and neutrophil infiltration in the pancreas (Figure 1B). In the PEG35-treated groups, only when the animals were treated prophylactically there were consistent reductions in pancreatic interstitial edema, leukocyte infiltration and acinar cell necrosis. Histological evaluation of the lungs showed significant edema, leukocyte infiltration and alveolar septal thickening (Figure 1C) associated with ANP. However, these findings were less marked when the animals were treated either prophylactically or therapeutically with PEG35.

### PEG35 abrogated ANP-induced IL6 expression in plasma

IL6 is an important multifunctional cytokine, which has many roles in inflammation, and its serum levels reflect the magnitude of the inflammatory response. This cytokine has been reported to have prognostic value for acute pancreatitis upon admission (Aoun E, Chen J, Reighard D, et al: Diagnostic accuracy of interleukin-6 and interleukin-8 in predicting severe acute pancreatitis: a meta-analysis. Pancreatology 2009; 9:777-85; and Gregoric P, Sijacki A, Stankovic S, et al: SIRS score on admission and initial concentration of IL-6 as severe acute pancreatitis outcome predictors. Hepatogastroenterology 2010; 57:349-53). It has been therefore measured its levels in plasma and, as expected, a significant increase was detected after ANP induction (Figure 2A). Prophylactic and therapeutic treatment with PEG35 resulted in a significant reduction on systemic IL6 levels.

### Prophylactic and therapeutic PEG35 ameliorated ANP-induced expression of pro-inflammatory cytokines in the lung

Next, it has been explored whether PEG35 administration might improve inflammatory response after ANP induction. To do so, it has been measured the gene expression of pro-inflammatory cytokines and chemokines (IL6, IL1β, TNFα and CXCL2, respectively) in both pancreas and lung. Pancreatic tissue levels of these mediators had risen markedly three hours after ANP induction compared with control animals (Figure 2B), except for TNFα. As expected, only prophylactic treatment with PEG35 was able to significantly reduce the ANP-induced increases in these cytokines. Regarding the inflammatory process in the lung, ANP induction raised expression levels of IL6, IL1β, TNFα and CXCL2 (Figure 2C). PEG35 administration prior to the induction of ANP significantly reduced IL6, IL1β and TNFα levels, and therapeutic administration significantly reduced the levels of IL6 and TNFα in the lung.

### PEG35 abrogated ANP-related adhesion molecules expression in the lung

The recruitment of leukocytes is a hallmark of inflammation. The process is controlled by complex interactions between surface receptors on neutrophils and their corresponding endothelial cell ligands (Ley K, Laudanna C, Cybulsky MI, et al: Getting to the site of inflammation: the leukocyte adhesion cascade updated. Nat Rev Immunol 2007; 7:678-89). To further study the protective function of PEG35 in ANP, it has been focused on the expression of two of the main adhesion molecules involved in this inflammatory disease: P-selectin and ICAM-1. A significant up-regulation in both adhesion molecules was evident in the pancreas and lung three hours after ANP induction compared to control-operated mice (Figure 3A and 3B). Only the prophylactic administration with PEG35 helped to reduce its expression in both these tissues, whereas therapeutic administration significantly lessened the gene expression of P-selectin and ICAM-1 in the lung.

### Effect of PEG35 treatment on plasma LDH activity

It has been next measured LDH activity in plasma. A significant increase in LDH activity occurred three hours after ANP induction (Figure 4). Both prophylactic and therapeutic use of PEG35 significantly reduced LDH activity.

### Prophylactic and therapeutic administration of PEG35 reduced the pulmonary neutrophil infiltration associated with ANP

Increased numbers of neutrophils (MPO positive cells) in both pancreas (Figure 5A) and lung (Figure 5B) marked the inflammatory response following ANP induction. Additionally, areas of intense cell infiltration with extravasation of leukocytes to the interacinar space were found. Pretreatment with PEG35 significantly attenuated the infiltration of leukocytes in the pancreas. In addition to local pancreatic neutrophil recruitment, a significant increase in MPO positive cells in the lung was noted in the ANP-induced animals compared to sham-operated mice. By contrast, both prophylactic and therapeutic intravenous administration of PEG35 lessened pulmonary neutrophil recruitment and extravasation.

## Claims

1. Polyethylene glycol (PEG) for use in the prevention of abdominal inflammatory disease and/or associated diseases.

2. PEG for the use according to claim 1, wherein PEG is PEG of at least 5kDa of molecular weight.

3. PEG for the use according to claim 2, wherein PEG is PEG35.

4. PEG for the use according to any of claims 1 to 3, wherein the abdominal inflammatory disease is acute or chronic pancreatitis.

5. PEG for the use according to claim 4, wherein the pancreatitis is acute necrotizing pancreatitis (ANP).

6. PEG for the use according to any of claims 1 to 3, wherein the associated disease is associated lung injury.

7. PEG for the use according to any of claims 1 to 6, **characterized in that** it is administered by intravenous administration.

8. PEG for the use according to any of claims 1 to 7, wherein the dose of PEG administered to a subject in need thereof is from about 0.01 mg/Kg to about 100 mg/Kg of body weight per day.

9. PEG for the use according to claim 6, wherein the dose of PEG administered to a subject in need thereof is a single dose.

10. A pharmaceutical composition which comprises a therapeutically effective amount of PEG and a pharmaceutically acceptable carrier for the use according to any of claims 1 to 9.


SEQUENCE LISTING

<110> CONSEJO SUPERIOR DE INVESTIGACIONES CIENTÍTICAS (CSIC)
NET ABRAHAM, MARC
OLIVE, GUILLAUME
LOPEZ, ALEXANDRE

<120> Polyethylene glycol for use in the prevention of abdominal
inflammatory diseases and/or associated diseases

<130> EP1641.1478

<160> 8

<170> PatentIn version 3.5

<210> 1
<211> 21
<212> DNA
<213> Artificial Sequence

<220>
<223> CXCL2 forward primer

<400> 1
tgctcaagac tccaaccact c 21

<210> 2
<211> 21
<212> DNA
<213> Artificial Sequence

<220>
<223> CXCL2 reverse primer

<400> 2
cacaacaacc cctgtaccct g 21

<210> 3
<211> 20
<212> DNA
<213> Artificial Sequence

<220>
<223> TNFalpha forward primer

<400> 3
atgggctccc tctcatcagt 20

<210> 4
<211> 20
<212> DNA
<213> Artificial Sequence

<220>
<223> TNFalpha reverse primer

<400> 4
gcttggtggt ttgctacgac 20

<210>  5
<211>  20
<212>  DNA
<213>  Artificial Sequence

<220>
<223>  P-Selectin forward primer

<400>  5
tctcctgcaa cgaggagttt                                                    20


<210>  6
<211>  20
<212>  DNA
<213>  Artificial Sequence

<220>
<223>  P-Selectine reverse primer

<400>  6
ggtgtcgaca ggacattgtg                                                    20


<210>  7
<211>  20
<212>  DNA
<213>  Artificial Sequence

<220>
<223>  ICAM-1 forward primer

<400>  7
gagcgacatt ggggaagaca                                                    20


<210>  8
<211>  21
<212>  DNA
<213>  Artificial Sequence

<220>
<223>  ICAM-1 reverse primer

<400>  8
cactcgctct gggaacgaat a                                                  21